(19) Europäisches Patentamt
European Patent Office
Office européen des brevets

(11) Publication number : **0 443 996 B1**

# (12) EUROPEAN PATENT SPECIFICATION

(45) Date of publication of patent specification : **22.06.94 Bulletin 94/25**

(51) Int. Cl.$^5$ : **C07C 219/22,** A61K 31/22, A61K 31/205

(21) Application number : **91830062.5**

(22) Date of filing : **21.02.91**

## (54) Esters of (R)(-)-carnitine and acyl (R)(-)-carnitines with beta-hydroxybutyric acid and pharmaceutical compositions containing them for inhibiting neuronal degeneration, liver proteolysis and for the treatment of coma.

(30) Priority : **23.02.90 IT 4766990**

(43) Date of publication of application : **28.08.91 Bulletin 91/35**

(45) Publication of the grant of the patent : **22.06.94 Bulletin 94/25**

(84) Designated Contracting States : **AT BE CH DE DK ES FR GB GR IT LI LU NL SE**

(56) References cited :
**EP-A- 0 167 115**
**DE-A- 3 015 636**
**GB-A- 2 071 091**

(73) Proprietor : **Sigma-Tau Industrie Farmaceutiche Riunite S.p.A.**
**Viale Shakespeare, 47**
**I-00144 Roma (IT)**

(72) Inventor : **Tinti, Maria Ornella**
**81, Via Ernesto Basile**
**I-00182 Rome (IT)**
Inventor : **Scafetta, Nazareno**
**10, Via Siena**
**I-00040 Pavona (RM) (IT)**
Inventor : **Misiti, Domenico**
**3, Via Bacchiglione**
**I-00199 Rome (IT)**

(74) Representative : **Fassi, Aldo**
**c/o Sigma-Tau**
**Industrie Farmaceutiche Riunite S.p.A.,**
**Viale Shakespeare 47**
**I-00144 Rome (IT)**

EP 0 443 996 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to the esters of R(-)-carnitine and acyl (R) (-)-carnitines with beta-hydroxybutyric acid in the form of their pharmacologically acceptable salts of formula (I)

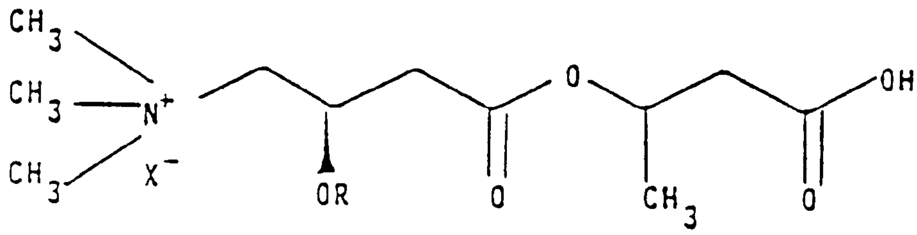

(I).

wherein $X^-$ is the anion of a pharmacologically acceptable acid e.g. chloride, bromide, orotate, acid aspartate, acid citrate, acid phosphate, acid fumarate, lactate, acid maleate, acid oxalate, acid sulfate and glucosephosphate, or in the form of inner salts of formula (I')

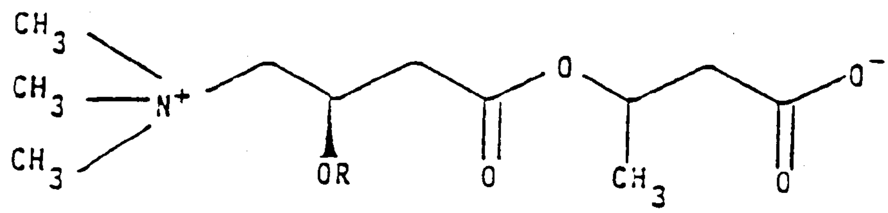

(I')

wherein R is a hydrogen or a straight or branched acyl group having from 2 to 5 carbon atoms, such as for instance acetyl, propionyl, n-butyryl, isobutyryl and isovaleryl.

These compounds are active in inhibiting neuronal degeneration (as it occurs in Alzheimer's senile dementia and Parkinson's disease) and liver proteolysis and in the treatment of coma.

The present invention also relates to orally or parenterally administrable pharmaceutical compositions for treating the foregoing pathologies, which comprise one of the compounds of formula (I) or(I') as active principle.

Esters of carnitine with hydroxy-substituted saturated organic acids (e.g. 2-hydroxybutyric, 2-hydroxy-2-methylbutyric and 2-methyl-3-hydroxypropionic acid) are known already; see e.g. US patent 4,766,222 assigned to Sigma-Tau Industrie Farmaceutiche Riunite S.p.A. These compounds, however, are O-esters (i.e. esters on the carnitine hydroxyl group) and endowed with pharmacological properties entirely different from and in no way related to the properties of the esters of the present invention.

Esters on the carnitine carboxyl group are described in Z. Physiol. Chem., 295, 377, 1953 and Z. Physiol. Chem., 346, 314, 1966. These are, however, esters of carnitine with aliphatic alcohols such as methanol, ethanol and butanol or with aromatic alcohols such as benzyl alcohol, not with hydroxyacids.

Also esters on both the hydroxyl and the carboxyl groups of carnitine are known, see e.g. DE-A-3015636. However, these compounds are not esters of beta-hydroxybutyric acid and moreover they are therapeutically useful for treating myocardial hypocontractility and as antidepressants in sleep disturbances. These pharmacological activities are totally unrelated to those of the present invention.

The non-limiting examples that follow show the preparation of the esters of acyl (R)(-)-carnitine chloride with beta-hydroxybutyric acid via the synthesis scheme which is illustrated hereinbelow.

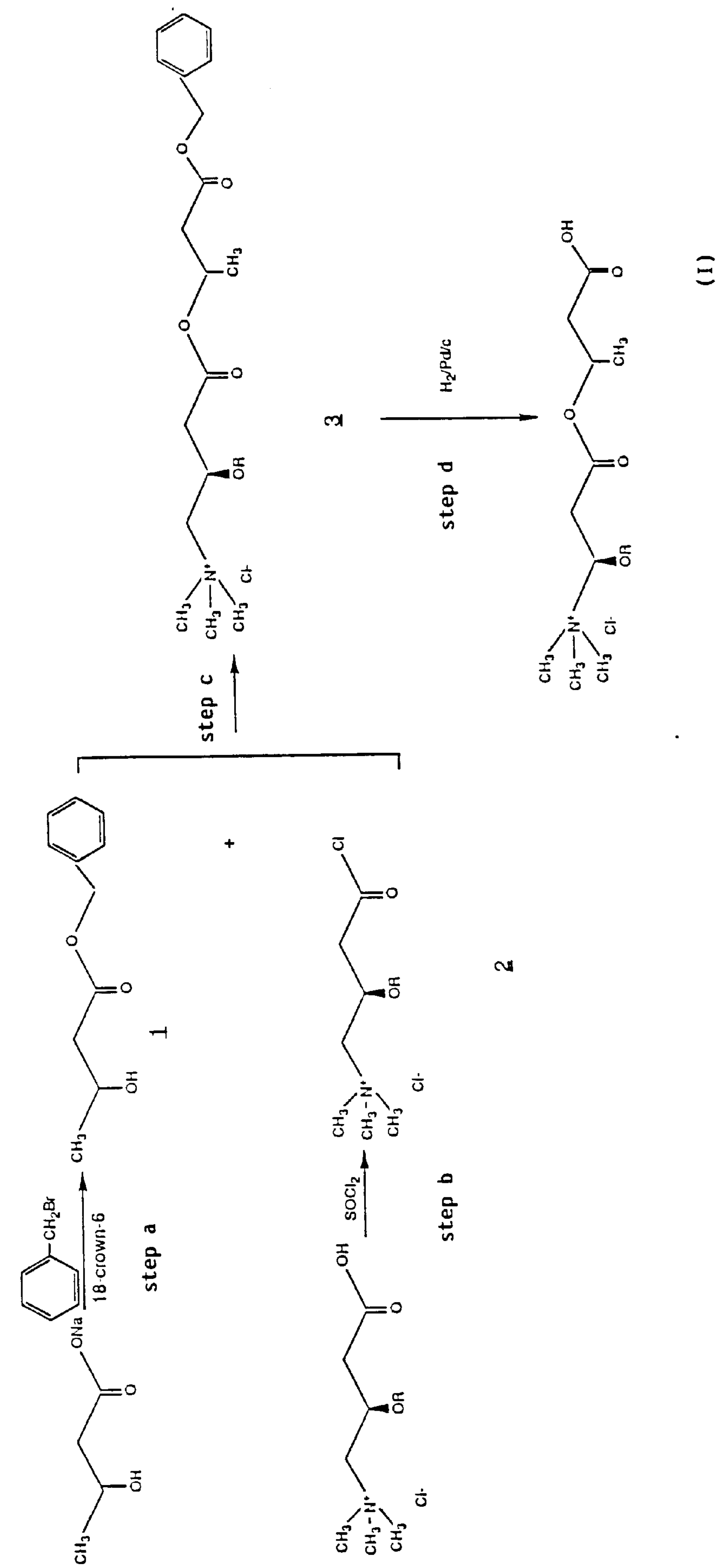


1

2

3

(I)

**Example 1**

Preparation of the ester of isovaleryl (R)(-)-carnitine chloride with (R,S)(±)-beta-hydroxybutyric acid (ST 687).

**Step a:**

Preparation of the benzyl ester of (R,S)(±)-beta-hydroxybutyric acid 1 (R,S)(±)-beta-hydroxybutyric acid sodium salt (1.2 g; 0.01 moles) was suspended in benzyl bromide (6 ml; 0.05 moles).
18 crown - 6 (0.264 g) dissolved in 7 ml acetonitrile was added to the mixture.
The solution was partially concentrated under a nitrogen stream and then kept under stirring at 80°C for 90 minutes. Following cooling, hexane - $H_2O$ was added. The separated and dried organic phase was concentrated and then distilled under vacuum to remove the excess of benzyl bromide.
A solid residue (1.1 g) was obtained which was identified as the title product, yield 56%, TLC $CHCl_3$9 - MetOH 1
Rf = 0.8
Gas chromatography column $HP_1$ 25 m; 0.32 mm ID; 0.33 μm film thickness
carrier (He) flow rate: 1 ml/min
Make up gas 40 ml/min
Splitting ratio 40 ml/min
Injector 220°C
Detector(Fid) 280°C
T column 120°C for 3 minutes, 15°C/min 250°C
Rt = 9.36 product 1
Rt = 4.84 benzylbromide absent
NMR $CDCl_3$ δ 7.3(5H,s,benzyl); 5.2(2H,s,$CH_2$-benzyl); 4.2(1 H,m,CH);
2.8(1H,s,broad OH); 2.5(2H,d,-$CH_2$COO); 1.2(3H,d,$CH_3$)

**Step b:**

Preparation of the acid chloride of isovaleryl R(-)-carnitine chloride 2 Thionyl chloride (7.7 ml; 0.1 moles) was added to isovaleryl (R)(-)-carnitine chloride (10 g; 0.035 moles).
The resulting mixture was kept at room temperature for 4 hours, then concentrated under vacuum to remove the excess of thionyl chloride. The residue was washed 3 times with anhydrous ethyl ether. The reaction raw product thus obtained was used in the following step without further purification.

**Step c:**

Preparation of the ester of isovaleryl (R)(-)-carnitine chloride with (R,S)(±) beta-hydroxybutyric acid benzyl ester 3.
Acid chloride of isovaleryl (R)( -)-carnitine chloride of step b (0.035 moles) was dissolved in 25 ml anhydrous tetrahydrofurane.
(R,S)(±) beta-hydroxybutyric acid benzyl ester (7 g; 0.035 moles) of step a was added to the solution.
The resulting reaction mixture was kept at 25°C under stirring overnight, then ethyl ether was added till complete precipitation. The solid product thus obtained was filtered and washed with ethyl ether. 14 g of product 3 were obtained. Yield 89%.

NMR $D_2O$ δ 5.7(5H,m,benzyl); 5.5(1H,m,-CH);
5.2(1H,m,COOCH); 5.0(2H,s,$CH_2$-benz.)
3.8(2H,m,$NCH_2$); 3.2(9H,s,$(CH_3)_3N+$);
2.8-2.5(4H,dd,$CH_2$-COOCH;$CH_2$COO) 2.2(2H,d,$OCOCH_2$)

1.8(1H,m, CH 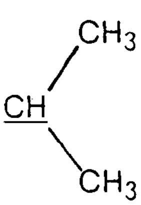 ); 1.2(3H,d,CH-$CH_3$); 0.8(6H,d,CH 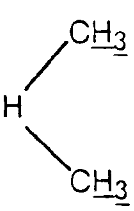 )

**Step d:**

Preparation of the ester of isovaleryl (R)(-)-carnitine chloride with (R,S)(±)-beta hydroxybutyric acid.
The product of step c (14 g; 0.031 moles) was dissolved in $H_2O$ - ethanol (1:1) (1000 ml) and then hydrogenated in the presence of 1.5 g 10% Pd/C at the pressure of 4 atmospheres for 2 hours.
The reaction mixture was filtered, concentrated to dryness under vacuum and the residue was crystallized from acetone-ethyl ether giving 10 g of a hygroscopic product.
TLC chloroform 4.2 Isopr OH 0.7 MeOH 2.8 $H_2O$ 1
AcOH 1.1 $R_F$ : 0.7
$[\alpha]_D^{25}$ = -21(c = 1, $H_2O$).

NMR $D_2O$ $\delta$ 5.7 (1H,m,CH); 5.3 (1H,m,-COOCH-);

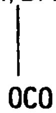

3.8(2H,m,$N^+CH_2$); 3.2 (9H,s,$(CH_3)_3N^+$);

2.8-(2H,d,$CH_2$-COO); 2.6(2H,d,$CH_2COOH$)

2.2(2H,d,$OCOCH_2$); 1.8(1H,m,CH 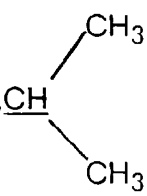 ); 1.2(3H,d,CH-$CH_3$);

0.8(6H,d,CH 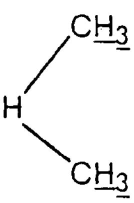 )

HPLC
Column μBondapack - $C_{18}$
Eluant $KH_2PO_4$ 0.05 M - $CH_3CN$ (85 -15)
UV detector λ = 205
Flowrate 1ml/min
Rt = 14-16 (the diastereomers are shown)

E.A. = $C_{15}H_{30}NO_6Cl$

| | C | H | N |
|---|---|---|---|
| calc. | 50.6 | 8.4 | 3.9 |
| found | 48.93 | 8.36 | 3.49 |

**Example 2**

Preparation of the ester of isobutyryl (R)(-)-carnitine chloride with (R,S)(±)beta-hydroxybutyric acid (ST 730)

**Step a**: same as in Example 1

**Step b**: same as in Example 1, except that isovaleryl (R)(-)-carnitine chloride was substituted by isobutyryl (R)(-)-carnitine chloride

**Step c**:

The intermediate 3, ester of isobutyryl (R)(-)-carnitine chloride with (R,S)(±)-beta-hydroxybutyric acid benzyl ester was purified via δ prep 300 preparative HPLC.
Column prepak $C_{18}$
Eluant $H_2O$-$CH_3CN$ 70-30
Flowrate 20 ml/min
Yield 50%

NMR $D_2O$ $\delta$ 7.5(5H,s,aromatic); 5.8(1H,m,-CH-); 5.3(m,1H,COOCH);

OCO CH3

5.1(2H,s,$CH_2$benz.); 4.0-3.8(2H,m,$\overset{+}{N}CH_2$); 3.2(9H,s$(CH_3)_3\overset{+}{N}$); 2.8(2H,m,

$CH_2COO$); 2.6(2H,m,$CH_2COOH$); 1.8(1H,m,OCOCH); 1.3(3H,d,CH-$CH_3$);

1.2(6H,d,C 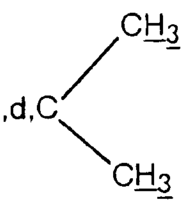 )

analytic HPLC
Column μ Bondapak $C_{18}$
Eluant phosphate buffer 0.05M-$CH_3CN$ 60-40
Flowrate 1 ml/min UV detector λ = 205 nm
Rt = 10.75

**Step d**

Ester of isobutyryl-(R)(-)-carnitine with (R,S)(±)beta-hydroxybutyric acid (ST 730).
Same as step d of Example 1.
$[\alpha]_D^{25}$ = -20.3(C = 1$H_2O$).
TLC $CHCl_3$ - $H_2O$-IsopOH - MeOH -AcOH
(4.2 - 1.05 - 0.7 - 2.8 - 1.05)

NMR $D_2O$ $\delta$ 5.7 (1H,m,-CH-); 5.25(1H,m,COOCH); 3.9-3.7(2H,m,$N^+CH_2$);
(the -CH- above is substituted by OCO; the COOCH above is substituted by $CH_3$)

3.2(9H,s$(CH_3)_3N^+$); 2.9(1H,m,$CH_2COO$); 2.7(2H,m,$\underline{CH_2}COOH$);

1.9(1H,m,OCOCH); 1.3(3H,d,CH-$\underline{CH_3}$); 1.1(6H,d,CH($C\underline{H_3}$)($C\underline{H_3}$))

| $C_{14}H_{28}NO_6Cl$ | | C | H | N | Cl |
|---|---|---|---|---|---|
| | calc. | 49.19 | 8.25 | 4.10 | 10.04 |
| | found | 50.26 | 8.12 | 3.59 | 10.61 |

HPLC
Column μ Bondapack - $C_{18}$
Eluant $KH_2PO_4$0.05M - $CH_3CN$ 85-15
UV detector $\lambda$ = 205 nm
Flowrate 1 ml/min
Rt = 8.10 - 9.98 (the two diastereomers are thus shown)

**Example 3**

Preparation of the ester of acetyl (R)(-)-carnitine chloride with (R,S)(±)beta-hydroxybutyric acid (ST 765)

Step a: same as in Example 1

Step b: same as in Example 1, except that isovaleryl (R)(-)-carnitine chloride was substituted by acetyl (R)(-)-carnitine chloride.

Step c:

Intermediate 3, ester of acetyl (R)(-)carnitine chloride with (R,S)(±)-beta-hydroxybutyric acid benzyl ester, was purified via preparative HPLC as described in step C of Example 2.
Yield 50%

NMR $D_2O$ $\delta$ 7.5(5H,s,aromatic); 5.7(1H,m,-CH-); 5.4-5.0(3H,m,s,COOCH-,
(the -CH- above is substituted by OCO)

$CH_2$-Ar); 3.8(2H,m,$N^+CH_2$); 3.2(9H,s,$(CH_3)_3N^+$); 2.8-2.5(4H,m,$COOCH_2$,

$CH_2COOH$) 2.2(3H,s,$COCH_3$); 1.4(3H,d,$CHC\underline{H_3}$)

analytic HPLC
Column μ Bondapack $C_{18}$

Eluant phosphate buffer $KH_2PO_4$ 0.05M - $CH_3CN$ 60-40
Flowrate 1 ml/min
UV detector $\lambda$ = 205 nm
Rt = 11.73

**Step d**:

Ester of acetyl (R)(-)-carnitine chloride with (R,S)(±)beta-hydroxybutyric acid (ST 765)
Prepared as described in step d of Example 1
$[\alpha]_D^{25}$ = -22.9 ($H_2O$ 1.2%)
TLC $CHCl_3$ - $H_2O$ - isoprOH - MetOH - AcOH (4.2 - 1.05 - 0.7 - 2.8 - 1.05)
Rf = 0.6

NMR $D_2O$ $\delta$ 5.7(1H,m,-CH-); 5.3(1H,m,COOCH); 3.9-3.7(2H,m,$N^+$-$CH_2$);
(the -CH- bearing OCO)
3.2(9H,s,$(CH_3)_3N^+$); 2.9(2H,m,$CH_2$COO); 2.7(2H,m,$CH_2$COOH)
2.2(3H,s,$COCH_3$); 1.4(3H,d,$CHCH_3$)

| E.A. $C_{13}H_{24}NO_6Cl$ | | C | H | N | Cl |
|---|---|---|---|---|---|
| | calc. | 47.90 | 7.42 | 4.29 | 10.88 |
| | found | 47.14 | 7.57 | 4.88 | 10.64 |

$H_2O$ 0.46%
HPLC
Column $\mu$ Bondapack $C_{18}$
Eluant phosphate buffer $KH_2PO_4$ 0.05M - $CH_3CN$ 90 - 10
Flowrate 0.5 ml/min
UV detector $\lambda$ = 205 nm
Rt = 11.68 - 12.83 (the two diastereomers are thus shown).

**Example 4**

Preparation of the ester of propionyl (R)(-)-carnitine chloride with (R,S)(±)beta-hydroxybutyric acid (ST 780).

**Step a**: same as in Example 1

**Step b**: same as in Example 1, except that isovaleryl R(-)carnitine chloride was substituted by propionyl R(-)carnitine chloride.

**Step c**:

Intermediate 3, ester of propionyl (R)(-)-carnitine chloride with (R,S)(±)-beta-hydroxybutyric acid benzyl ester, was purified via preparative HPLC as described in step c of example 2.
Yield 50%

CH3

NMR $CDCl_3$ δ 7.3(5H,s,aromatic); 5.6(1H,m,-CH-); 5.3(1H,m,C<u>H</u>);

OCO

5.1(2H,s,$CH_2$-Ar);4.0(2H,m,$N^+CH_2$); 3.4(3H,s,$(CH_3)_3N^+$); 2.9-2.5(4H,mC<u>H</u>$_2$CO OCH; C<u>H</u>$_2$COOH); 2.3(2H,t,$OCOCH_2$); 1.4-1.0(6H,m,$CH_2$<u>CH</u>$_3$;<u>CH</u>$_3$CH)

analytic HPLC
Column μ Bondapack $C_{18}$

| | |
|---|---|
| Eluant phosphate buffer 0.005 M | 60 |
| Acetonitrile | 40 |

Flowrate 1 ml/min
UV detector λ = 205 nm
Rt = 8.46

**<u>Step d</u>:**

Ester of propionyl-(R)(-)-carnitine chloride with (R,S)(±)beta-hydroxybutyric acid (ST 780).
Prepared as described in step d of Example 1.
$[\alpha]_D^{25}$ = -23.9(C=1% $H_2O$)

NMR $D_2O$ δ 5.6(1H,m,CH); 5.3(1H,q,CH); 3.8(2H,m,$N^+CH_2$); 3.2(9H,s,$(CH_3)_3$

OCO CH3

$N^+$); 2.9-2.4 (4H,d,d,C<u>H</u>$_2$COOCH;C<u>H</u>$_2$COOH); 1.3(3H,t,$CH_2$C<u>H</u>$_3$); 1.0(3H,d,CH)

HPLC
Column μ Bondapack $C_{18}$

| | |
|---|---|
| Eluant phosphate buffer $KH_2PO_4$ 0.005 M | 90 |
| $CH_3CN$ | 10 |

Flowrate 0.5 ml/min
UV detector λ = 20.5 nm
Rt = 6.40-7.07 (the two diastereomers are thus shown).

**Example 5**

Preparation of the ester of R(-)-carnitine chloride with R,S(±)beta-hydroxybutyric acid (ST 784). The compound was prepared as described in the previous Examples 1-4.
$[\alpha]_D^{25}$ = -11.1 (C = 1% $H_2O$)

NMR $D_2O$ δ 5.3(1H,m,COOCH); 4.6(1H,m,$\underline{CH}$); 3.4(2H,dd,$N^+CH_2$);

OH

3.2(9H,s,$(CH_3)_3N^+$); 2.7(4H,m,$\underline{CH_2}$COOCH; $\underline{CH_2}$COOH);

1.3 (3H,d,CH-$\underline{CH_3}$)

HPLC
Column Novapak $C_{18}$
mobile phase $KH_2PO_4$ 50 mM
Flowrate 1 ml/min
$R_t$ = 4.56-5.01 min (the two diastereomers are thus shown)

**Example 6**

Ester of isobutyryl (R)(-)-carnitine chloride with R(-)-beta-hydroxybutyric acid (ST 863).
The compound was prepared as described in Example 2 (ST 730)
The compound of step c, ester of isobutyryl (R)(-)-carnitine with R(-)-beta-hydroxybutyric acid benzyl ester, showed the following characteristics:
$[\alpha]_D^{25}$ = - 11.1 (C = 1% MetOH)
HPLC
Column μ Bondapack $C_{18}$
mobile phase $NaClO_4$ 0.05M-$CH_3CN$ (60-40)
Flowrate 1.5 ml/min
UV detector λ = 205 nm
$R_t$ = 15.64 min
The compound of step d, i.e. the title compound ester of isobutyryl (R)(-)-carnitine chloride with R(-)-beta-hydroxybutyric acid (ST 863), showed the following characteristics:
$[\alpha]_D^{25}$ = -11.6 (C = 1% $H_2O$)
HPLC
Column μ Bondapack $C_{18}$
mobile phase $KH_2PO_4$ 0.05M - $CH_3CN$ 70-30
Flowrate 1 ml/min
UV detector λ = 205 nm
$R_t$ = 8.25

**Example 7**

Ester of isobutyryl (R)( -)-carnitine chloride with S(+)-beta-hydroxybutyric acid (ST 864).
The compound was prepared as described in Example 2 (ST 730)
The compound of step c, ester of isobutyryl (R)(-)-carnitine chloride with S(+)-beta-hydroxybutyric acid benzyl ester, showed the following characteristics:
$[\alpha]_D^{25}$ = -15.4 (C = 1% MetOH)
HPLC
Column μ Bondapak $C_{18}$
mobile phase $NaClO_4$ 0.05M-$CH_3CN$ (60-40)

Flowrate 1.5 ml/min
UV detector λ = 205 nm
$R_t$ = 14.79 min
The compound of step d, i.e. the title compound ester of isobutyryl (R)(-)-carnitine with S(+)-beta-hydroxybutyric acid (ST 864), showed the following characteristics:
$[\alpha]_D^{25}$ = - 21.7 (C = 1% $H_2O$)
HPLC
Column μ Bondapack $C_{18}$
mobile phase $KH_2PO_4$ 0.05M-$CH_3CN$ (70-30)
Flowrate 1 ml/min
UV detector λ = 205 nm
$R_t$ = 7.32 min

**Example 8**

Ester of butyryl (R)(-)-carnitine chloride with (R,S)(±)-betahydroxybutyric acid (ST 877).
The compound was prepared as described in Example 1.
The compound of step c, ester of butyryl (R)(-)-carnitine chloride with (R,S)(±)-betahydroxybutyric acid benzyl ester, showed the following characteristics:
$[\alpha]_D^{25}$ = -12.8 (C = 1% $H_2O$)
HPLC
Column 53 ODS1 (100 mm x 1 mm) Sperisorb
mobile phase $KH_2PO_4$ 0.05M - $CH_3CN$ 70-30
UV detector λ = 205 nm
Flowrate 0.1 ml/min
$R_t$ = 30 min

NMR $D_2O$ δ 7.5(5H,s,benzyl); 5.6(1H,m,CH);
OCO
5.2(3H,s+m,$CH_2$-benzyl; $\underline{CH}$-$CH_3$);
3.7(2H,m,$N^+CH_2$-); 3.3(9H,s,$(CH_3)_3N^+$-);
2.8(4H,m,$CH_2COO$,$OCOCH_2$); 2.4(2H,t,$\underline{CH_2}COOCH_2$);
1.7(2H,q,$\underline{CH_2}CH_3$); 1.2(3H,d,$CH\underline{CH_3}$);
1.0(3H,t,$CH_2\underline{CH_3}$)

The compound of step d, i.e. the title compound ester of butyryl (R)(-)-carnitine chloride with (R,S)(±)-beta-hydroxybutyric acid (ST 877), showed the following characteristics:
$[\alpha]_D^{25}$ = -18.9 (C = 1% $H_2O$)
HPCL
Column Bondapak NH2
mobile phase $KH_2PO_4$ 0.05M - $CH_3CN$ 35-65
UV detector λ = 205 nm
Flowrate 0.1 ml/min
$R_t$ = 5.62

NMR $D_2O$ $\delta$ 5.6(1H,m CH-); 5.2(1H,m,C<u>H</u>); 3.8(2H,m,$N^+CH_2$)

OCO $CH_3$

3.2(9H,s,$(CH_3)_3N^+$); 2.8(4H,m,$CH_2COO$,$OCOCH_2$);

2.4(2H,t,<u>$CH_2$</u>COOH); 1.7(2H,m,<u>$CH_2$</u>$CH_3$);

1.2(3H,d,CH<u>$CH_3$</u>); 1.0(3H,t,$CH_2$<u>$CH_3$</u>)

Effect of ST 687 on the neurologic deficit, memory impairment and cerebral oedema in post-oligaemic rats.

The study was conducted with a view to assessing the therapeutical effect of ST 687 administered i.p. to rats immediately after the effect of a transient forebrain oligohaemia in the experiment animals were detected. In particular, the propensity of post-oligaemic rats to develop conditioning was studied in a one-trial passive avoidance task. Concurrently, the neurologic deficit during a 3-day period following the ischaemic insult was assessed, and finally the extent of the oedema was assessed by measuring the water content of the cerebral tissue.

The effects of ST 687 were compared with those of acetyl (R)(-)-carnitine studied under identical experimental conditions.

In the experiments Sprague-Dawley (Iffa Credo) male rats weighing 230-250 g were used, that had been caged (5 rats/cage) under conditions of controlled temperature (22°C±1°C), 50% relative humidity and 12-hours dark-light cycle (light on from 8 a.m. to 8 p.m.). The rats were fed UAR (Epinay Orge, France) laboratory chow and had free access to tap water. The rats were caged for 5 days before surgery.

Under light ether anesthesia, carotid arteries were isolated and loosely surrounded by a thread. Twenty-four hours later, reversible incomplete forebrain ischemia was produced by bilateral common carotid artery occlusion combined with sodium nitroprusside-induced arterial hypotension (1.1 mg/rat s.c.). Mean arterial blood pressure (MABP) was lowered and maintained nearby 6.6 kPa for 45 min. Then it gruadually returned to normal within the 60th minute when carotid occlusion was removed.

The neurological deficit was assessed via the observational method described by Irwin S.: Comprehensive observational assessment: I a. A systematic, qualitative procedure for assessing the behavioural and physiologic states of the mouse. Psychopharmacologia (Berl.), 1968, 13 : 222-257, for quantifying the behavioural and physiologic state of the mouse. The rats were lifted vertically by mid-tail approximately 15 cm above a rod and lowered to elicit the visual placing response, usually characterized by an extension of forelimbs before contact. The rating was as follows: 3 = normal behaviour (the rat grasps the rod) : 2 = mild anterolateral rotation of the forelimbs (the grasping reflex only occurs when the rat is placed close to the rod) : 1 = severe rotation of the forelimbs and of the body (the grasping reflex occurs occasionally when the rat touches the rod) : 0 = no grasping reflex. The neurological deficit according to this criterion was evaluated respectively 3, 24, 48 and 72 hours following oligohaemia.

The functional aspects of the cerebral ischemic injury were assessed by a one trial learning procedure (passive avoidance reaction) as originally described by Kurtz K. H. e Pearl J.: The effects of prior fear experience in acquired drive learning. J. Comp. Physiol, Psychol, 1960, 53 : 201-206, and more extensively developed by Buresova et al.: Effect of atropine on learning, extinction, retention and retrieval in rats. Psychopharmacologia, 1964, 5 : 255-263.

Four hours after clipping-off untreated post-oligaemic rats were placed into an apparatus consisting of a large illuminated compartment (40 x 40 cm) connected by an opening to a small dark compartment (10 x 10 cm) with an electrified grid floor. The animals placed into the large compartment were allowed to explore the apparatus for three minutes. The latency to enter and the time spent in the small compartment were measured with a stop watch. Habituation to the experimental conditions was repeated 24 and 29 hours after ischemia. At the end of the third habituation trial, the opening between the two compartments was closed and the rat, placed into the small compartment, received intermittent electrical foot-shocks for one minute. The retention of the passive avoidance towards the small compartment was tested 24 and 48 hours after the last habituation trial i.e. 53 and 77 hours post-oligohaemia, respectively. The criterion used to determine whether an animal was conditioned was based upon the rat remaining in the large illuminated compartment for 180 sec. without entry into the small dark compartment.

Immediately after the last retention trial, i.e. 77 hours post-oligohaemia, rats were sacrificed by decapitation, their brains rapidly removed and macroscopically examined in terms of swelling. Brain water content was determined by the method wet weight/dry weight.

Death-rate, cerebral oedema and passive avoidance in post-oligaemic rats and following treatment with acetyl(R)(-)-carnitine and ST 687

| | Dose mg. kg i.p. twice a day | n.1 | n.2 | Death rate after 72 hours (%) | Incidence of cerebral oedema upon excision (3) | Incidence of retention of conditioned response (%) | |
|---|---|---|---|---|---|---|---|
| | | | | | | + 53 h | + 77 h |
| Oligohaemia | 0 | 19 | 9 | 52,6 | 66,6 | 44,4 | 33,3 |
| Acetyl (R)(-)-carnitine | 12,5 | 15 | 10 | 33,3 | 80,0 | 80,0 | 80,0 |
| | 25,0 | 17 | 10 | 41,2 | 60,0 | 60,0 | 70,0 |
| | 50,0 | 20 | 10 | 50,0 | 90,0 | 70,0 | 50,0 |
| ST 687 | 2.5 | 19 | 10 | 47,4 | 60,0 | 20,0 | 40,0 |
| | 5.0 | 16 | 10 | 37,5 | 50,0 | 60,0 | 60,0 |
| | 7,5 | 16 | 6 | 62,5 | 66,7 | 53,3 | 66,7 |

n 1 = number of oligaemic rats

n 2 = number of survivors

n 3 = number of rats exhibiting cerebral oedema

$p < 0,05$ according to the continuity-corrected chi$^2$ test.

Protective effect of ST 784 against acetaminophen (paracetamol)-induced hepatic damage.

Paracetamol has been widely used as analgesic and antipyretic. Paracetamol overdosage is known to provoke serious hepatic damages.

Male Wistar rats weighing 200-250 g (15 rats/group) that had been keept fasting for at least 12 hours, were administered a single dose of paracetamol (1 g/kg body weight, per os). 100 g paracetamol were dissolved in 1000 ml of 5% (w/v) carboxymethylcellulose suspension in water. (Hence, the animals were actually administered 10 ml paracetamol solution/kg body weight). 101 mg ST 784/kg body weight were administered orally (as aqueous solution) 1, 8 and 24 hours, respectively, following paracetamol administration. The animals were sacrificed 32 hours following paracetamol administration.

Transaminases (SGOT and SGPT) were measured in blood serum. ST 784 provoked a decrease in transaminases exceeding 60% ($p \leqq 5$) with respect to the control animals.

The compounds of the present invention are orally or parenterally administered, in any of the usual pharmaceutical forms which are prepared by conventional procedures well-known to those persons skilled in the pharmaceutical techonology. These forms include solid and liquid oral unit dosage forms such as tablets, capsules, solution, syrups and the like as well as injectable forms, such as sterile solutions for ampoules and phials.

For these pharmaceutical forms the usual solvents, diluents and excipients are used. Optionally, sweetening, flavouring and preservative agents can also be bresent. Non limiting examples of such agents are sodium carboxymethylcellulose, polysorbate, mannitol, sorbitol, starch, avicel, talcum and other agents which will be apparent to those skilled in the pharmaceutical technology.

The dose which is administered will be determined by the attending physician having regard to the age, weight and general conditions of the patient, utilizing sound professional judgement. Although effective results can be noticed at doses as low as 5 to 8 mg/kg of body weight daily, a dose of from about 10 to about 50 mg/kg of body weight is preferred. Whenever necessary, larger doses can be safely administered in view of the low toxicity of the compounds of this invention.

As non-limiting examples and depending on the specific pharmaceutical form of administration, the following dosages can be indicated:

| | |
|---|---|
| for the phials | : from 5 to 500 mg |
| for the capsules | : from 15 to 50 mg |
| for the tablets | : from 15 to 500 mg |
| for the oral solution | : from 15 to 50 mg |

## Claims

### Claims for the following Contracting States : AT, BE, CH, DE, DK, FR, GB, IT, LI, LU, NL, SE

1. Esters of (R)(-)-carnitine and acyl (R)(-)-carnitines with betahydroxybutyric acid, in the form of pharmacologically acceptable salts of formula (I)

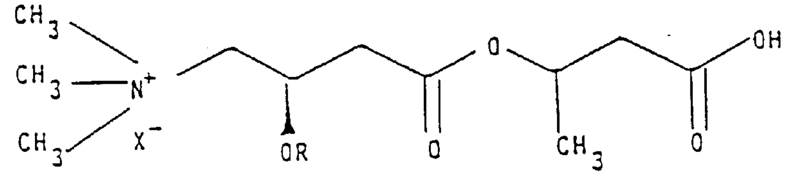

(I)

wherein $X^-$ is the anion of a pharmacologically acceptable acid and R is hydrogen or a straight or branched acyl group having from 2 to 5 carbon atoms.

2. Esters of (R)(-)-carnitine and acyl (R)(-)-carnitines with betahydroxybutyric acid in the form of inner salts of formula (I')

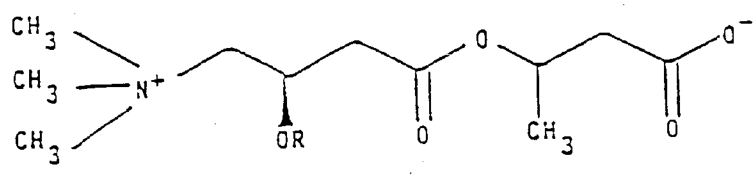

(I')

wherein R is hydrogen or a straight or branched acyl group having from 2 to 5 carbon atoms.

3. Esters according to claims 1 or 2, wherein R is selected from hydrogen, acetyl, propionyl, n-butyryl, isobutyryl and isovaleryl.

4. Esters according to claim 1, wherein $X^-$ is selected from chloride, bromide, orotate, acid aspartate, acid citrate, acid phosphate, acid fumarate, lactate, acid maleate, acid oxalate, acid sulfate and glucosephosphate.

5. An orally or parenterally administrable composition comprising an ester of formula (I) or (I') as defined in claims 1 or 2 as active principle.

6. An orally or parenterally administrable composition for inhibiting neuronal degeneration, liver proteolysis and for the treatment of coma comprising and ester of formula (I) or (I') as defined in claims 1 or 2 as active principle and a pharmacologically acceptable excipient therefor.

7. Composition according to claim 6, in unit dosage form, comprising between 5 and 500 mg of an ester of formula (I) or (I').

**Claim for the following Contracting States : ES, GR**

1. A process for preparing esters of (R)(-)-carnitine and acyl (R)(-)-carnitine wiht β-hydroxybutyric acid of general formula (I)

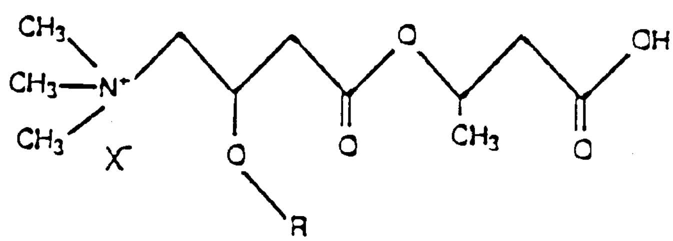

(I)

wherein $X^-$ is the anion of a pharmacologically acceptable acid and R is H or a straight or branched acyl group having from 2 to 5 carbon atoms, comprising:

1) condensing the sodium salt of β-hydroxybutyric acid with benzyl chloride in the presence of crown-ethers, in an organic solvent, in an inert gas atmosphere, at a temperature comprised between 20°C and 30°C, for 1-2 hours, and isolating the benzylester of β-hydroxybutyric acid thus obtained via distillation under vacuum;

2) condensing the acid chloride of (R)(-)-carnitine or acyl (R)(-)-carnitine with the benzylester of β-hydroxybutyric acid in an inert anhydrous organic solvent, at a temperature comprised between 20°C and 30°C, for 12-24 hours, and isolating the compound thus obtained, acyl (R)(-)-carnitine ester with β-hydroxybutyric acid and benzylester, from the reaction mixture by precipitation with an organic solvent, such as ethyl ether or acetonitrile; and

3) hydrogenating the compound obtained in step 2) in a water or ethanol solution or mixtures thereof,

in the presence of a hydrogenation catalyst, such as 5% or 10% Pd/C, for 30-180 minutes, at a hydrogen pressure of 2.026-5.066 bars, and isolating the product thus obtained, acyl (R)(-)-carnitine β-hydroxybutyric acid, by concentrating under vacuum the solution to dryness.

## Patentansprüche

### Patentansprüche für folgende Vertragsstaaten : AT, BE, CH, DE, DK, FR, GB, IT, LI, LU, NL, SE

1. Ester von (R)(-)-Carnitin und Acyl(R)(-)-carnitinen mit beta-Hydroxybuttersäure in der Form von pharmakologisch akzeptablen Salzen der Formel (I)

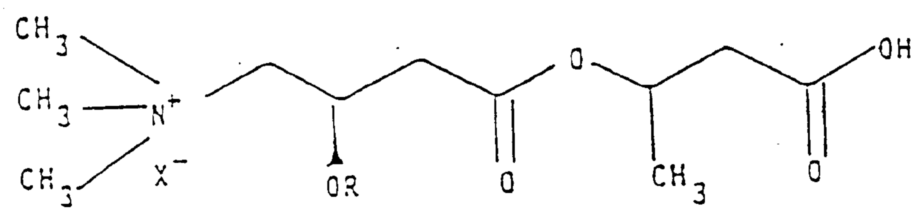

(I)

worin $X^-$ das Anion einer pharmakologisch akzeptablen Säure und R Wasserstoff oder eine geradkettige oder verzweigte Acylgruppe mit 2 bis 5 Kohlenstoffatomen ist.

2. Ester von (R)(-)-Carnitin und Acyl(R) (-)-carnitinen mit beta-Hydroxybuttersäure in der Form von inneren Salzen der Formel (I')

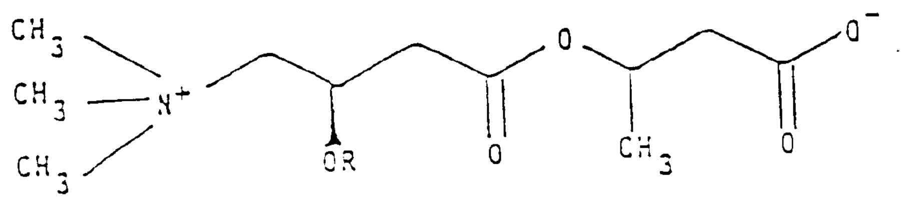

(I')

worin R Wasserstoff oder eine geradkettige oder verzweigte Acylgruppe mit 2 bis 5 Kohlenstoffatomen ist.

3. Ester nach Anspruch 1 oder 2, worin R aus Wasserstoff, Acetyl, Propionyl, n-Butyryl, Isobutyryl und Isovaleryl ausgewählt ist.

4. Ester nach Anspruch 1, worin $X^-$ aus Chlorid, Bromid, Orotat, Säureaspartat, Säurecitrat, Säurephosphat, Säurefurmarat, Laktat, Säuremaleat, Säureoxalat, Säuresulfat und Glukosephosphat ausgewählt ist.

5. Oral oder parenteral verabreichbare Zusammensetzung, umfassend einen Ester der Formel (I) oder (I') nach Anspruch 1 oder 2 als Hauptbestandteil.

6. Oral oder parenteral verabreichbare Zusammensetzung zum Inhibieren von neuronaler Degenerierung, Leberproteolyse und zur Behandlung von Koma, umfassend einen Ester der Formel (I) oder (I') nach Anspruch 1 oder 2 als Hauptbestandteil und einen pharmakologisch akzeptablen Exzipienten dafür.

7. Zusammensetzung nach Anspruch 6, in Einheitsdosierungsform, umfassend zwischen 5 und 500 mg eines Esters der Formel (I) oder (I').

**Patentanspruch für folgende Vertragsstaaten : ES, GR**

1. Verfahren zur Herstellung von Estern von (R) (-)-Carnitin und Acyl(R)(-)-carnitinen mit beta-Hydroxybuttersäure der allgemeinen Formel (I)

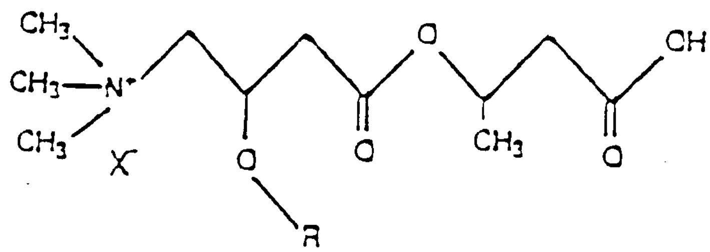

(I)

worin $X^-$ das Anion einer pharmakologisch akzeptablen Säure ist und R H oder eine geradkettige oder verzweigte Acylgruppe mit 2 bis 5 Kohlenstoffatomen ist, umfassend:
1) Kondensieren des Natriumsalzes von beta-Hydroxybuttersäure mit Benzylchlorid in der Gegenwart von Kronen-Ethern in einem organischen Lösungsmittel in einer Inertgas-Atmosphäre bei einer Temperatur zwischen 20 und 30°C für 1 bis 2 Stunden und Isolieren des somit erhaltenen Benzylesters von beta-Hydroxybuttersäure durch Destillation unter Vakuum;
2) Kondensieren des Säurechlorides von (R) (-)-Carnitin oder Acyl(R) (-)-carnitin mit dem Benzylester von beta-Hydroxybuttersäure in einem inerten wasserfreien organischen Lösungsmittel bei einer Temperatur zwischen 20 und 30°C für 12 bis 24 Stunden und Isolieren der somit erhaltenen Verbindung, Acyl(R) (-)-carnitinester mit beta-Hydroxybuttersäure und Benzylester, aus der Reaktionsmischung durch Ausfällung mit einem organisches Lösungsmittel wie Ethylether oder Acetonitril; und
3) Hydrieren der in Schritt 2) erhaltenen Verbindung in einer Wasser- oder Ethanollösung oder Mischungen davon in der Gegenwart eines Hydrierungskatalysators wie 5 % oder 10 % Pd/C für 30 bis 180 Minuten bei einem Wasserstoffdruck von 2,026 bis 5,066 Bar und Isolieren des somit erhaltenen Produktes, Acyl (R)(-)-carnitin-beta-hydroxybuttersäure, durch Konzentrieren der Lösung unter Vakuum bis zur Trockene.

## Revendications

**Revendications pour les Etats contractants suivants : AT, BE, CH, DE, DK, FR, GB, IT, LI, LU, NL, SE**

1. Esters de R(-)-carnitine et d'acyl-(R)(-)-carnitines avec l'acide bêta-hydroxybutyrique, sous forme de leurs sels pharmacologiquement acceptables de formule (I)

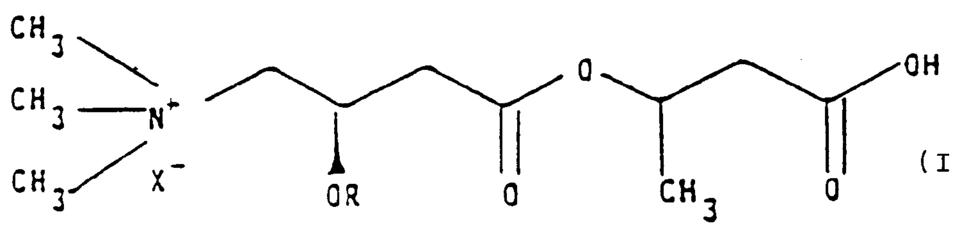

(I)

dans laquelle $X^-$ est l'anion d'un acide pharmacologiquement acceptable et R est un atome d'hydrogène ou un groupe acyle linéaire ou ramifié comportant de 2 à 5 atomes de carbone.

2. Esters de R(-)-carnitine et d'acyl-(R)(-)-carnitines avec l'acide bêta-hydroxybutyrique, sous forme de sels internes de formule (I')

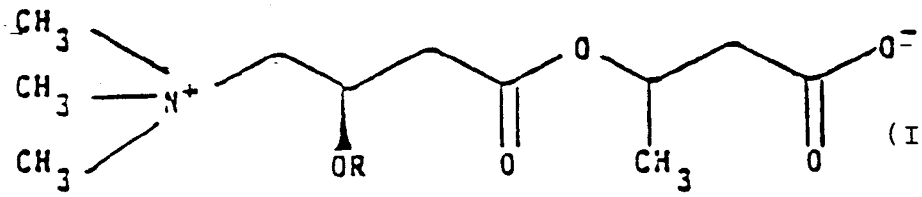

(I')

dans laquelle R est un hydrogène ou un groupe acyle linéaire ou ramifié comportant de 2 à 5 atomes de carbone.

**3.** Esters selon les revendications 1 ou 2, dans lesquelles R est choisi parmi un atome d'hydrogène et un groupe acétyle, propionyle, n-butyryle, isobutyryle et isovaléryle.

**4.** Esters selon la revendication 1, dans lesquels $X^-$ est choisi parmi un chlorure, un bromure, un orotate, un aspartate acide, un citrate acide, un phosphate acide, un fumarate acide, un lactate acide, un maléate acide, un oxalate acide, un sulfate acide et un phosphate de glucose.

**5.** Composition administrable par voie orale ou parentérale comprenant un ester de formule (I) ou (I') tels que définis dans les revendications 1 ou 2, comme principe actif.

**6.** Composition administrable par voie orale ou parentérale pour inhiber la dégénérescence neuronale, la protéolyse du foie et pour le traitement du coma, comprenant un ester de formule (I) ou (I') tel que défini dans les revendications 1 ou 2, comme principe actif et un excipent pharmacologiquement acceptable pour ce dernier.

**7.** Composition selon la revendication 6, sous forme posologique unitaire, comprenant entre 5 et 500 mg d'un ester de formule (I) ou (I').

**Revendication pour les Etats contractants suivants : ES, GR**

**1.** Procédé de préparation d'esters de R(-)-carnitine et d'acyl-(R)(-)-carnitines avec l'acide bêta-hydroxybutyrique, sous forme de leurs sels pharmacologiguement acceptables de formule (I)

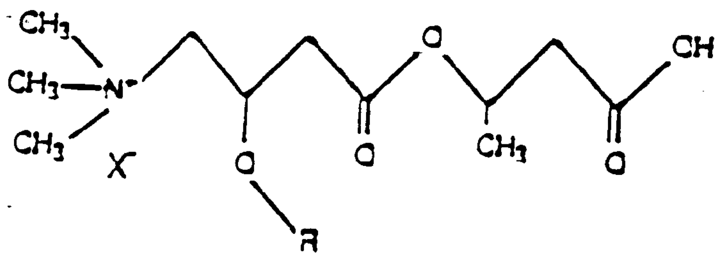

(I)

dans laquelle $X^-$ est l'anion d'un acide pharmacologiquement acceptable et R est un atome d'hydrogène ou un groupe acyle linéaire ou ramifié comportant de 2 à 5 atomes de carbone, comprenant :

1) La condensation du sel de sodium d'acide bêta-hydroxybutyrique avec du chlorure de benzyle, en présence d'éther couronne, dans un solvant organique, sous une atmosphère de gaz inerte, à une température comprise entre 20°C et 30°C, pendant 1-2 heures, et l'isolement de l'ester benzylique d'acide bêta-hydroxybutyrique ainsi obtenu par distillation sous vide.

2) La condensation du chlorure d'acide de (R)(-)-carnitine ou d'acyl-(R)(-)-carnitine avec l'ester benzylique d'acide bêta-hydroxybutyrique, dans un solvant organique anhydre inerte, à une température comprise entre 20°C et 30°C, pendant 12-24 heures, et l'isolement du composé ainsi obtenu, à savoir l'ester d'acyl-(R)(-)-carnitine avec l'acide bêta-hydroxybutyrique et l'ester benzylique, du mélange réactionnel par précipitation avec un solvant organique comme l'éther diéthylique ou l'acétonitrile ; et

3) l'hydrogénation du composé obtenu dans l'étape 2), dans une solution dans l'eau ou dans l'éthanol, ou des mélanges de celles-ci, en présence d'un catalyseur d'hydrogénation, comme du Pd/C à 5% ou à 10%, pendant 30-180 minutes, sous une pression d'hydrogène de 2,026-5,066 bars, et l'isolement du produit ainsi obtenu, à savoir l'acide acyl-(R)(-)-carnitine bêta-hydroxybutyrique, par concentration sous vide de la solution à sec.